# EUROPEAN PATENT APPLICATION

(11) **EP 1 834 666 A2**
(43) Date of publication of application: **19.09.2007**
(21) Application number: 07251051.4
(22) Date of filing: 14.03.2007
(51) Int. Cl.: A61N 1/375

(54) **Integrated filter feedthrough assemblies made from low temperature co-fired (LTCC) tape**

(30) Priority: 14.03.2006 US 782120
(71) Applicant: Greatbatch Ltd., Clarence, NY 14031 (US)
(72) Inventor: Fu, Richard, Ellicott City, MD 21042 (US); Zhu, Mingguang, East Amherst, NY 14051 (US); Frysz, Christine A., Orchard Park, NY 14127 (US); Billings, Kenneth, Lancaster, NY 14086 (US)
(74) Representative: Duckett, Anthony Joseph

(57) **Abstract**

A filter capacitor comprising a substrate of at least one layer of a low temperature co-fires ceramic (LTCC) tape supporting alternating active and ground electrode layers segregated by a dielectric layer is described. The substrate is preferably a laminate of three LTCC tapes pieces that are heated under pressure and at a relatively low temperature to become a laminate that maintains its shape and structure dimensions even after undergoing numerous sintering steps. Consequently, relatively thin active and ground electrode layers along with the intermediate dielectric layer can be laid down or deposited on the LTCC substrate by a screen-printing technique. A second laminate of LTCC tapes is positioned on top of the active/dielectric/ground layers to finish the capacitor. Consequently, a significant amount of space is saved in comparison to a comparably rated capacitor or, a capacitor of a higher rating can be provided in the same size as a conventional prior art capacitor.

## Description

### CROSS-REFERENCE TO RELATED APPLICATION

This application claims priority from provisional application Serial No. 60/782,120, filed March 14, 2006.

### BACKGROUND OF THE INVENTION

### 1. Field of the Invention

This invention relates generally to simplified feedthrough filter capacitor assemblies and related methods of construction. Feedthrough filter capacitor assemblies are used to decouple and shield undesirable electromagnetic interference (EMI) signals from implantable medical devices and other electronic devices.

More specifically, this invention relates to simplified and reduced cost filter capacitors for ceramic feedthrough terminal pin assemblies. In the present invention, the filter capacitor assembly is mounted to the ceramic feedthrough terminal pin assembly. The ceramic feedthrough is used to connect a terminal pin or electrode through a hermetically sealed housing to internal electronic components of the medical device while the filter capacitor decouples EMI signals against entry into the sealed housing via the terminal pin. This invention is particularly designed for use in cardiac pacemakers (bradycardia devices), cardioverter defibrillators (tachycardia), neurostimulators, internal drug pumps, cochlear implants and other medical implant applications. This invention is also applicable to a wide range of other EMI filter applications, such as military or space electronic modules, where it is desirable to preclude entry of EMI signals into a hermetically sealed housing containing sensitive electronic circuitry.

In that respect, feedthrough terminal pin assemblies are generally well known in the arts for connecting electrical signals through the housing or case of an electronic instrument. For example, in implantable medical devices such as cardiac pacemakers, defibrillators, and the like, the feedthrough assembly comprises one or more conductive terminal pins supported by an insulator structure for feedthrough passage from the exterior to the interior of the medical device. Many different insulator structures and related mounting methods are known in the art for use in medical devices wherein the insulator structure provides a hermetic seal to prevent entry of body fluids into the housing thereof. However, the feedthrough terminal pins are typically connected to one or more lead wires leading to a body organ such as a heart. The lead wires effectively act as an antenna and tend to collect stray EMI signals for transmission into the interior of the medical device. That is why hermetic feedthrough assemblies are combined with a ceramic feedthrough filter capacitor to decouple interference signals to the medical device housing. However, a primary feature of the simplified feedthrough filter capacitor described herein is volume reduction without compromising effectiveness and reliability. This is accomplished by elimination of some of the volume previously occupied by the capacitor dielectric using new screen printing techniques. The present feedthrough filter capacitor is also less costly to manufacture than a comparably rated prior art capacitor.

### 2. Prior Art

In a typical prior art unipolar construction for a feedthrough filter capacitor, such as described in U.S. Pat. No. 5,333,095 to Stevenson et al., a round/discoidal (or rectangular) ceramic feedthrough filter capacitor is combined with a hermetic feedthrough terminal pin assembly. In use, the coaxial capacitor permits passage of relatively low frequency electrical signals along the terminal pin while shielding and decoupling/attenuating undesired interference signals of relatively high frequency to the conductive housing of the medical device.

One type of hermetic feedthrough terminal pin subassembly widely used in implantable medical devices employs an alumina ceramic insulator which, after sputtering/metallization procedures, is gold brazed into a titanium ferrule. In addition, there are terminal pins, typically made of platinum, which are also gold brazed to the alumina ceramic insulator to complete the hermetic seal. See for example, the subassemblies disclosed in U.S. Pat. Nos. 3,920,888 to Barr; 4,152,540 to Duncan et al.; 4,421,947 to Kyle and 4,424,551 to Stevenson et al. Separately, the feedthrough filter capacitor is constructed by preassembly of the coaxial capacitor and then mounting it onto or within the cylindrical or rectangular hermetically sealed feedthrough terminal pin subassembly including the conductive pins and ferrule.

The feedthrough capacitor is of a coaxial construction having two sets of electrode plates embedded in spaced relation within an insulative dielectric substrate or base. The dielectric base is typically formed as a ceramic monolithic structure. One set of "active" electrode plates is electrically connected at an inner diameter cylindrical surface to a terminal pin in a unipolar (one terminal pin) construction. Feedthrough capacitors are also available in bipolar (two), tripolar (three), quadpolar (four), pentapolar (five), hexpolar (six), and additional terminal pin configurations. The inner active plates are coupled in parallel together by a metallized layer which is either glass frit fired or plated onto the ceramic capacitor. This metallized band, in turn, is mechanically and electrically coupled to the terminal pin by a conductive adhesive or soldering, and the like.

The other or second set of "ground" electrode plates is coupled at an outer diameter surface of the discoidal capacitor to a cylindrical ferrule of conductive material. The outer ground plates are coupled in parallel together by a metallized layer which is fired, sputtered or plated onto the ceramic capacitor. This metallized band, in turn, is coupled to the ferrule by conductive adhesive, soldering, brazing, welding, and the like. The ferrule is electrically connected in turn to the conductive housing of the electronic device.

The device housing is constructed from a biocompatible metal such as of a titanium alloy, which is electrically and mechanically coupled to the hermetic feedthrough terminal pin subassembly, which is, in turn, electrically coupled to the feedthrough filter capacitor. As a result, the filter capacitor coupled to the feedthrough terminal pin subassembly prevents entrance of interference signals to the interior of the device housing, where such interference signals could otherwise adversely affect the desired device function such as cardiac pacing or defibrillation.

Although feedthrough filter capacitor assemblies of the type described perform in a generally satisfactory manner, the associated manufacturing and assembly costs are unacceptably high. One area where costs can be reduced is in the manufacture of the feedthrough filter capacitor.

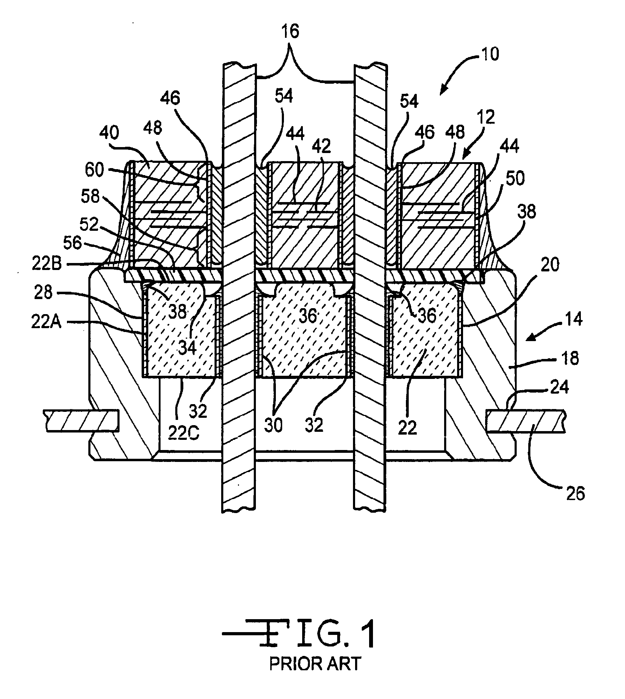

Fig. 1 illustrates a typical prior art feedthrough filter capacitor assembly 10 comprising a filter capacitor 12 mounted to a feedthrough terminal pin subassembly 14. The filter feedthrough capacitor assembly 10 is shown in one preferred form comprising a so-called bipolar configuration having two separate conductive terminal pins 16 extending through the discoidal-shaped filter capacitor 12 and feedthrough terminal pin subassembly 14.

The feedthrough terminal pin subassembly 14 comprises a ferrule 18 defining an insulator-receiving bore 20 surrounding an insulator 22. Suitable electrically conductive materials for the ferrule substrate 18 include titanium, tantalum, niobium, stainless steel or combinations of alloys thereof. Titanium is preferred for the ferrule 18, which may be of any geometry, nonlimiting examples being round, rectangle, and oblong. A surrounding inwardly facing annular channel 24 is provided in the ferrule 18 to facilitate attachment of the feedthrough filter capacitor assembly 10 to the casing 26 of, for example, the implantable medical device. The method of attachment may be by laser welding or other suitable methods.

The insulator 22 is of a ceramic material such as of alumina, zirconia, zirconia toughened alumina, aluminum nitride, boron nitride, silicon carbide, glass or combinations thereof. Preferably, the insulating material 22 is alumina, which is highly purified aluminum oxide, and comprises a sidewall 22A extending to a first upper surface 22B and a second lower surface 22C. A layer of metal 28, referred to as metallization, is applied to the sidewall 22A of the insulating material 22 to aid in the creation of a brazed hermetic seal. Suitable metallization materials 28 include titanium, niobium, tantalum, gold, palladium, molybdenum, silver, platinum, copper, carbon, carbon nitride, titanium nitrides, titanium carbide, iridium, iridium oxide, tantalum, tantalum oxide, ruthenium, ruthenium oxide, zirconium, and mixtures thereof. The metallization layer 28 may be applied by various means including, but not limited to, sputtering, e-beam deposition, pulsed laser deposition, plating, electroless plating, chemical vapor deposition, vacuum evaporation, thick film application methods, aerosol spray deposition, and thin cladding.

The insulator 22 has a sufficient number of bores 30 to receive the terminal pins 16. The inner surfaces of these bores 30 are provided with a metallization layer 32 in a similar manner as the previously described insulator sidewall 22A. The terminal pins 16 are then received in the bores 30. Preforms (not shown) of a conductive, biostable material, such as gold or gold alloy, are moved over the terminal pins 16 to rest against the upper insulator surface 22B adjacent to annular notches 34 in the insulator. Similarly, a gold preform (not shown) is positioned at the junction of the ferrule insulator-receiving bore 20 and the upper insulator surface 22B. The thusly-assembled feedthrough terminal pin assembly 14 is then heated in an oven or furnace to melt the preforms and cause them to form their respective brazes 36 and 38. Braze 36 hermetically seals the terminal pins 16 to the insulator 22 at the terminal pin bores 30 while braze 38 hermetically seals the insulator 22 to the ferrule 18 at the insulator-receiving bore 20.

The feedthrough filter capacitor 12 comprises a dielectric 40 formed from multiple layers of a tape cast ceramic or ceramic-based material containing multiple capacitor-forming conductive first "active" electrode layers 42 and second "ground" electrode layers 44 screen-printed in an alternating manner on top of the tape cast dielectric. This layered assembly is then sintered to provide a monolithic dielectric body containing the electrode layers 42, 44. Although the exemplary drawing shows in exaggerated scale a pair of the active electrode layers 42 in parallel staggered relation with a corresponding pair of the ground electrode layers 44, it will be understood that a large plurality of typically 5 to 40 conductive layers 42 can be provided in alternating stacked and parallel spaced relation with a corresponding number of the conductive layers 44.

Each of the active electrode layers 42 is subdivided into two spaced-apart and generally pie-shaped electrode plates (not shown). Accordingly, the two electrode plates 42 of each layer group are electrically insulated from each other by the dielectric material 40 of the capacitor 12. The multiple spaced-apart layers of the active electrode plates 42 are formed in stacked alignment with the respective active electrode plates 42 of overlying and underlying layers to define two respective active plate stacks. The two terminal pins 16 pass generally centrally through respective bores 46 formed in these active plate stacks, and are conductively coupled to the associated stacked set of active electrode plates 42 by a suitable conductive surface lining such as a surface metallization layer 48 lining each bore 46.

A plurality of spaced-apart layers of the second or "ground" electrode plates 44 are also formed within the capacitor 12. The ground electrode plates 44 are in stacked relation alternating or interleaved with the layers of active electrode plates 42. These ground electrode plates 44 include outer perimeter edges which are exposed at the outer periphery of the dielectric body 18 where they are electrically connected in parallel by a suitable conductive surface such as a surface metallization layer 50. Importantly, however, the outer edges of the active electrode plates 42 terminate in spaced relation with the outer periphery of the capacitor body 12. In that manner, the active electrode plates 42 are electrically isolated by the dielectric body 40 from the conductive layer 50 coupled to the ground electrode plates 44. Similarly, the ground electrode plates 44 have inner edges which terminate in spaced relation with the terminal pin bores 46, whereby the ground electrode plates 44 are electrically isolated by the dielectric body 40 from the terminal pins 16 and the conductive metallization layer 48 lining the pin bores 46. The number of active and ground electrode plates 42 and 44, together with the dielectric 18 thicknesses or spacing there between may vary in accordance with the desired capacitance value and voltage rating.

The feedthrough capacitor assembly 10 is constructed by moving the filter capacitor 12 over the feedthrough terminal pin subassembly 14. A non-conductive disk-shaped member 52 is positioned about the terminal pins 16 at a location sandwiched between the upper insulator surface 22B of the feedthrough terminal pin subassembly 14 and the bottom of the capacitor terminal pin bores 46. In this position, the non-conductive disc 52 supports the capacitor 12 in spaced relation above the insulating material 22. The metallized surface 48 within the terminal pin bores 46 is then connected electrically to the terminal pins 16 by means of a conductive adhesive bead 54, or by soldering or brazing or the like. In a preferred form, the conductive adhesive 54 is applied to the annular gap between the pins 16 and the capacitor metallized surface 48, and allowed to fill a portion (about one-half) of the gap length. Similarly, the metallized surface 50 associated with the ground electrode plates 44 of the capacitor 12 is connected electrically to the ferrule 18 by means of an additional fillet 56 of conductive adhesive or the like. One preferred conductive adhesive comprises a curable polyimide adhesive loaded with conductive particles such as spheres or flakes, as described by way of example in U.S. Pat. No. 4,424,551 to Stevenson et al., which is incorporated by reference herein. However, it will be understood that other conductive connecting means may be used, such as solder, braze or the like. Importantly, the adhesive beads 54, 56 establish an electrically conductive mounting of the capacitor 12 in a secured stable manner to the feedthrough terminal pin assembly 14.

Mechanically, the barium titanate material typically used as the capacitor dielectric material 40 is relatively weak and prone to fracture. Also, if the dielectric material 40 is not sufficiently thick, it tends to warp into a "potato chip" shape upon being heated during sintering of the tape cast material. That is why in the prior art filter feedthrough capacitor assembly 10, the thickness of the respective lower and upper dielectric zones 58 and 60 below and above the intermediate zone occupied by the electrode layers 42, 44 is from about 0.007 inches to 0.015 inches, preferably about 0.010 inches. In other words, the thickness from the lower surface of the dielectric 18 adjacent to the non-conductive disc 52 to the lowest ground plate 44 is about 0.010 inches and the distance from the upper active plate 42 to the upper surface of the dielectric body 18 also about 0.010 inches. Depending on the number of active and ground electrode plates and the spacing between them, the intermediate zone in the dielectric body supporting the electrode plates 42, 44 is typically a minimum of about 0.010 inches, to much more. Therefore, the capacitor 12 is generally of a minimum thickness of about 0.030 inches and in certain applications can be significantly greater. The bulk of this thickness is occupied by the lower and upper dielectric zones 58, 60 sandwiching the intermediate zone of the electrode plates and each being about 0.010 inches thick. If these dielectric zones 58, 60 could be made thinner without compromising function and reliability including planarity, significant size reductions could be realized.

Accordingly, there is a need for a novel feedthrough filter capacitor assembly that addresses the drawbacks noted above in connection with the prior art. In particular, a novel capacitor assembly is needed which significantly reduces the volume occupied by this assembly, or is of a comparable volume, but of a significantly higher capacitance rating, without any diminution in filtering performance or reliability and yet that may be utilized in many of the same applications where such subassemblies are now found. Additionally, the improved feedthrough filter capacitor assembly should lend itself to standard manufacturing processes so that cost reductions can be realized immediately. Of course, the new design must be capable of effectively filtering out undesirable electromagnetic interference (EMI) signals from the target device. The present invention fulfills these needs and provides other related advantages.

### SUMMARY OF THE INVENTION

In a preferred form, a feedthrough terminal pin filter assembly according to the present invention comprises an outer ferrule hermetically sealed to an alumina insulator or a fused glass dielectric material seated within the ferrule. The insulator or dielectric material is also hermetically sealed to at least one terminal pin. That way, the feedthrough assembly prevents leakage of fluid, such as body fluid in a human implant application, past the hermetic seal at the insulator/ferrule and insulator/terminal pin interfaces.

When used in an implanted medical device, the feedthrough terminal pins are connected to one or more lead wires which sense signals from the patient's heart and also couple electronic pacing pulses from the medical device to the heart. Unfortunately, these lead wires can act as an antenna to collect stray EMI signals for transmission via the terminal pins into the interior of the medical device. Such unwanted EMI signals can disrupt proper operation of the medical device, resulting in malfunction or failure. To prevent unwanted EMI signals from transmitting via the terminal pins into the interior of the medical device, a filter capacitor is mounted on the ferrule.

According to the present invention, the filter capacitor comprises at least one active electrode layer (plate) and at least one ground electrode layer (plate) physically segregated from each other by a dielectric. The dielectric and the electrode layers are screen printed on a substrate comprising one of more layers of a relatively low temperature co-fired ceramic (LTCC) tape. The filter capacitor is completed with a LTCC cap placed on top of the active electrode/dielectric/ground electrode subassembly.

The use of a LTCC tape substrate and a LTCC tape cap in conjunction with screen printing of the various layers comprising the capacitor means that the total thickness of the capacitor is significantly less than that of a conventionally constructed filter capacitor. Since these capacitor devices are intended for use in applications where a premium is placed on space, such as in implantable medical devices, and the like, providing a filter capacitor of reduced size represents a significant advancement in the art. This is done without compromising the capacitor's effectiveness in attenuating unwanted EMI signals or its voltage rating.

These and other objects and advantages of the present invention will become increasingly more apparent by a reading of the following description in conjunction with the appended drawings.

### BRIEF DESCRIPTION OF THE DRAWINGS

Fig. 1 is a cross-sectional view of a prior art filter feedthrough capacitor assembly 10.
Fig. 2 is a plan view of a plurality of capacitors 100 supported on a LTCC substrate 102 according to the present invention.
Fig. 3 is a cross-sectional view taken along line 3-3 of Fig. 2.
Fig. 4 is a partial cross-sectional view of the filter capacitor shown in Fig. 3 electrically connected to a terminal pin 116 by a conductive epoxy bead 118.
Fig. 5 is a partial cross-sectional view of the filter capacitor shown in Fig. 3 electrically connected to a terminal pin 116 by a metallization layer 117 and conductive epoxy 119.
Fig. 6 is a cross-sectional view of another embodiment of a filter capacitor 120 according to the present invention.
Figs. 7 and 8 are cross-sectional views of various embodiment of the filter capacitor 120 shown in Fig. 6 attached to a hermetic feedthrough.
Fig. 9 is a cross-sectional view of another embodiment of a filter feedthrough capacitor 300 with the ground electrode 302 being the first layer screen-printed on the LTCC substrate 102 instead of the active layer 104 as in Fig. 3.
Fig. 10 is a cross-sectional view of another embodiment of a filter feedthrough capacitor 400 with the dielectric layers extending completely to the terminal pin bore 102 and the outer casing edge 108 to thereby segregate the active layers from each other and the ground layers from each other.

### DETAILED DESCRIPTION OF THE PREFERRED EMBODIMENTS

As used herein, the term "low temperature" is defined as a heating that occurs at less than about 950°C.

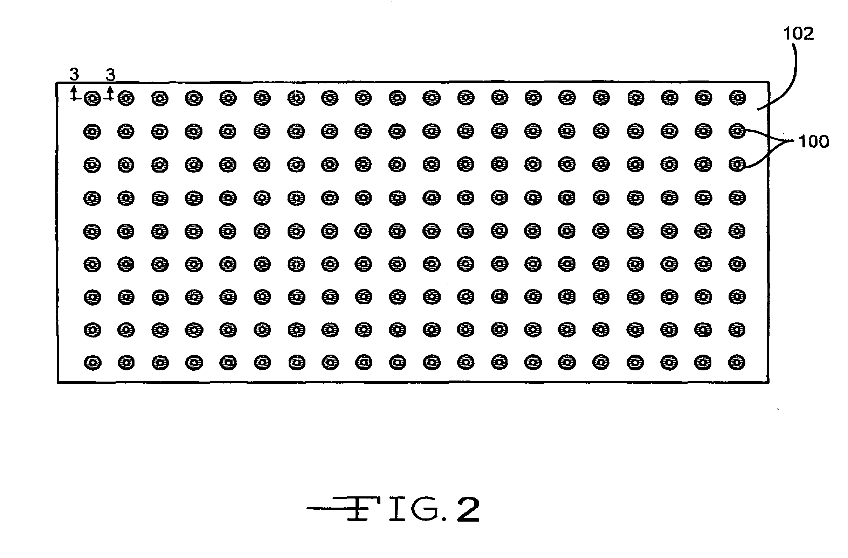

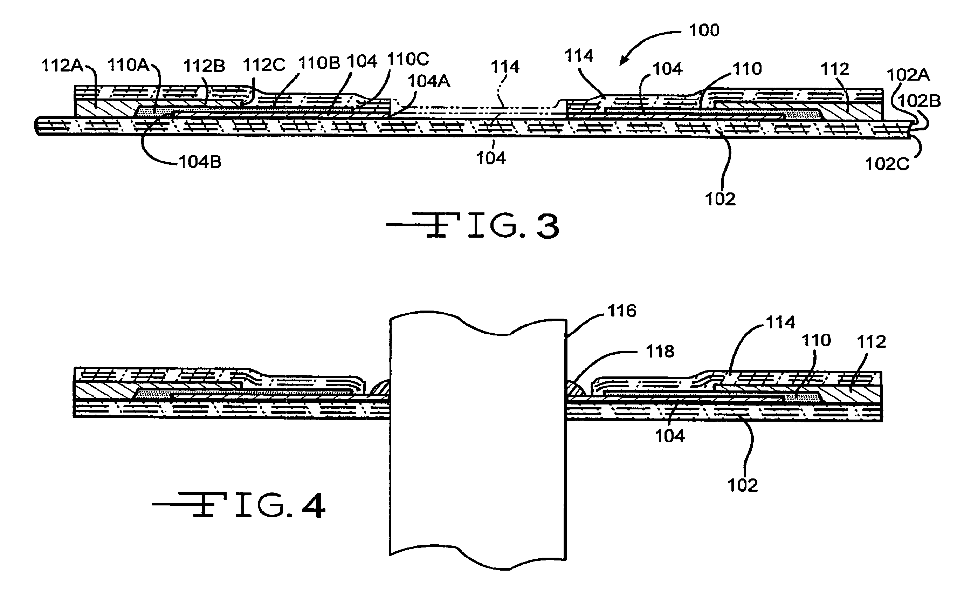

Referring now to the drawings, a preferred manufacturing process for constructing a filter capacitor 100 according to the present invention is shown in Figs. 3, 4 and 6. The process includes a screen-printing machine (not shown) that accurately and precisely prints the various layers of the filter capacitor 100 on an electrically non-conductive substrate 102 comprising one or more layers of a relatively low temperature co-fired ceramic (LTCC) tape. Fig. 2 illustrates one completed capacitor substrate 102 comprising 180 individual filter capacitor structures printed thereon in nine rows of twenty capacitors 100.

In the exemplary filter capacitor 100, the substrate 102 is comprised of three layers 102A, 102B and 102C of the LTCC tape. Low temperature, co-fired ceramics are made of a ceramic oxide powder that is mixed with various organic materials, such as acrylic resins, for example a synthetic resin commercially available from E. I. Du Pont De Nemours & Co. under the designation Elvacite^{®}, and glasses such as borosilicate and, possibly, a dielectric material. This mixture is cast or coated in sheets on a carrier where it is allowed to dry, after which it is cut into tape strips. Such strips may have a thickness of between 4½ mils to about 12 mils when dry.

Suitable ceramic materials are selected from the group consisting of alumina, zirconia, aluminum nitride, boron nitride, and silicon carbide. Borosilicate is a sintering aid that lowers the temperature at which a dense ceramic can be achieved. Suitable dielectric materials are of the titanate group, preferably barium strontium titanate and sodium bismuth titanate. Finally, the organic binder is preferably cellulose based. Suitable solvents include terpineol (boiling point = 220°C), butyl carbitol (b.p. = 230°C), cyclohexanone (b.p. = 155.6°C), n-octyl alcohol (b.p. = 171°C), ethylene glycol (b.p. = 197°C), glycerol (b.p. = 290°C) and water. These are relatively high boiling point solvents that do not evaporate at room temperature and maintain rheology or viscosity during the manufacturing process.

The "green tape" sheets 102A, 102B and 102C are then stacked, aligned and subjected to isostatic pressure of about 3,000 psi for about 10 minutes at from about 65°C to about 85°C. The thermo-plastic organic materials in the tape sheets soften and, under the pressure, flow together to effectively mechanically weld the individual sheets into a monolithic structure or a single laminated part. The thusly formed laminate is dried in an oven at from about 100°C to about 250°C to remove the binder and then cut into the appropriate shape to form the "green" substrate 102 (Fig. 2). The substrate 102 is much like a single thick slab and may have a total thickness of about 0.003 inches to about 0.050 inches. Examples of low temperature co-fired ceramic tape processing can be found in "Development of a Low Temperature Co-fired Multi-layer Ceramic Technology," by William A. Vitriol et al., 1983 ISHM Proceedings, and pages 593-598.

Fig. 3 shows that a representative one of the filter capacitors 100 is built by first screen-printing a bottom conductive layer 104 directly on the LTCC substrate 102. Layer 104 is screen-printed to cover the area that will eventually become the terminal pin bore 106. This is done for ease of manufacturing. In its final form, layer 104 comprises an inner, proximal edge 104A extending from the bore 106 to a distal edge 104B, spaced from an outer edge 108 of the capacitor. The terminal pin bore 106 is shown in phantom because it is preferably cut out of the LTCC substrate 102 later in the manufacturing process although, if desired, the bore 106 can be provided before any screen printing takes place. Similarly, the outer edge 108 of the capacitor 100 is shown in phantom because it is preferably cut into the LTCC substrate 102 at completion of the screen printing process, but that is not necessary. A preferred cutting method is by laser cutting. While shown in cross-section, it should be understood that the bottom active electrode layer extends 360° about the circumference of the terminal pin bore 106. Suitable active materials include Au-, Pt-, Cu-, Ni-, Ir-, Pd-, Ta-based pastes, the preferred one being an Ag-Pt based paste.

Preferably, the bottom active electrode layer 104 consists of two sub-layers, although one or more than two can be used if desired. After each active sub-layer is printed, the LTCC substrate 102 is put into a belt fed infrared oven to dry-out or flash-off any remaining solvent in the printed pattern.

After the requisite number of active sub-layers are printed and dried, a dielectric layer 110 is screen-printed over the bottom active electrode layer 104. The dielectric layer 110 has a proximal base portion 110A supported directly on the LTCC substrate 102 immediately adjacent to the outer edge 104B of the bottom active layer 104. This base portion 110A does not extend completely to the outer edge 108 of the capacitor 100. Instead, the proximal dielectric base portion 110A leads to a distal planar portion 110B that is in direct contact with the upper surface of the bottom active layer 104. The distal dielectric portion 110B extends to an edge 110C that ends spaced from the terminal pin bore 106. The dielectric material can be a BaTiO₃ based thick film paste with a relatively high dielectric constant of about 10,000 k. Other useful high dielectric materials are barium strontium titanate and sodium bismuth titanate, among others. Preferably, the dielectric layer 110 consists of two sub-layers, although one or more than two can be used if desired. After each dielectric sub-layer is printed, the subassembly is put into the belt fed infrared oven to flash-off any remaining solvent in the printed pattern.

In an alternate embodiment, the dielectric layer is not screen-printed. Instead it is a laminate of the LTCC tape that is placed on top of the active electrode layer 104. As is the case with the substrate 102, if the dielectric layer is a LTCC tape laminate, it preferably has three layers. In this case, the thusly processed subassembly is subjected to isostatic pressure of about 3,000 psi for about 10 minutes at from about 65°C to about 85°C.

After the requisite number of dielectric sub-layers is printed and dried, a ground electrode layer 112 is screen printed on the LTCC substrate 102. The ground electrode layer 112 is of a similar material as the active electrode layer 104 and comprises a base portion 112A supported directly on the LTCC substrate 102 immediately adjacent to the outer edge 108 of the capacitor 100. The proximal ground base portion 112A leads to a distal planar portion 112B that is in direct contact with the upper surface of the dielectric layer 110. The distal ground electrode portion 112B extends to an edge 112C spaced from the terminal pin bore 106, but directly vertically above the active electrode layer 104. The ground electrode 112 is of a similar material as the active electrode layer 104 and is dried after each sub-layer in a similar manner as the active electrode layer 104. Each active and ground electrode layer has a thickness of about 0.0004 inches to about 0.0008 inches. In a similar manner as the active electrode layer 104, the dielectric layer 110 and the ground electrode layer 112 each extends 360° about the circumference of the terminal pin bore 106.

The capacitor 100 may be finished by a cap layer of an LTCC tape laminate 114 extending to the outer substrate edge 108, but in the capacitor's finished form covering from the outer capacitor edge 108 to the terminal pin bore 106. The cap LTCC layer 114 is of materials similar to the LTCC substrate 102. The capacitor assembly consisting of the LTCC substrate 102/active electrode layer 104/dielectric layer 110/ground electrode layer 112/cap LTCC layer 114 is then subjected to an isostatic pressing at about 3,000 psi for about 10 minutes at from about 65°C to about 85°C.

The thusly formed plurality of capacitors are individually punched or otherwise cut from the substrate 102 of Fig. 2 and subjected to a final sintering at about 700°C to about 950°C for about 10 to 30 minutes. As is the case with the LTCC substrate 102, the cap LTCC layer 114 preferably consists of three layer of tape, although more or less can be used, if desired.

Thus, Fig. 3 illustrates a filter capacitor 100 comprising one active electrode layer 104 and one ground electrode layer 112 segregated from each other by an intermediate dielectric layer 110 sandwiched between the LTCC substrate layer 102 and the LTCC cap layer 114. This structure is sufficient to provide a feedthrough filter capacitor 100 according to the present invention. What is noteworthy is that the filter capacitor 100 is from about 30% to about 50% thinner (the distance from the bottom of substrate 102 to the top of cap 114) than the prior art capacitor 12 of Fig. 1 while providing comparable capacitance and being structurally stable.

Fig. 4 shows the finished capacitor 100 electrically connected to a terminal pin 116 by a conductive epoxy bead 118. The terminal pin 116 is part of a hermetic feedthrough assembly as previously described with respect to the prior art filter feedthrough capacitor 10. For the sake of simplicity the feedthrough is not shown in this drawing. Nonetheless, the epoxy bead 118 surrounds the terminal pin 116 of a feedthrough assembly and is in direct electrical contact with the active electrode layer 104 and the terminal pin.

While the active and ground layers 104, 112 have been described as a single layer that is not necessary. As described in U.S. Patent No. 5,978,204 to Stevenson, each layer 104, 112 can comprise two closely spaced apart layers separated from each other by a relatively thin dielectric layer. This patent is assigned to the assignee of the present invention, and incorporated herein by reference.

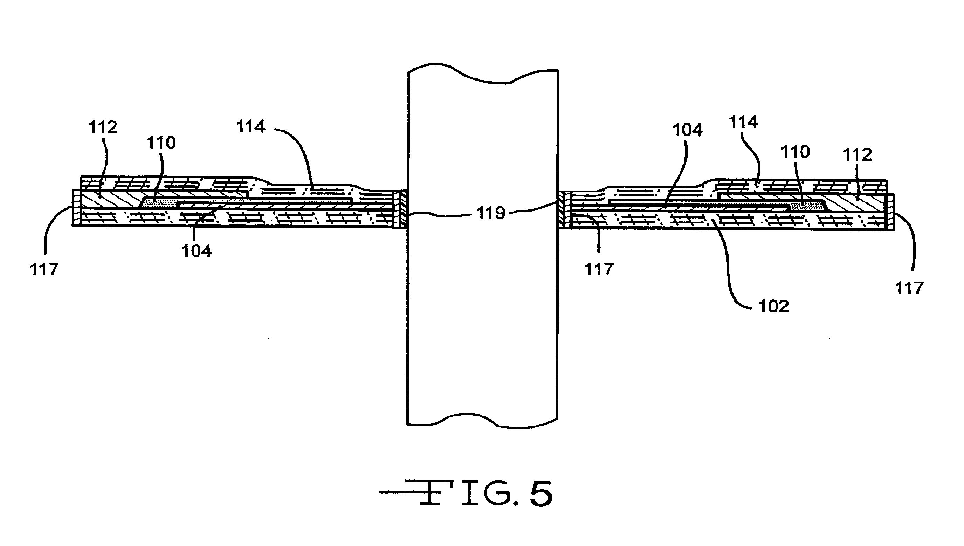

Fig. 5 shows the filter capacitor 100 of Fig. 3, but provided with a metallization layer 117 applied to the sidewall of the terminal pin bore 106 and the outer edge 108. As with the prior art capacitor 10, suitable metallization materials 117 include titanium, niobium, tantalum, gold, palladium, molybdenum, silver, platinum, copper, carbon, iridium, iridium oxide, ruthenium, ruthenium oxide, zirconium, and mixtures thereof. The metallization layer 117 may be applied by various means including, but not limited to, sputtering, e-beam deposition, pulsed laser deposition, plating, electroless plating, chemical vapor deposition, vacuum evaporation, thick film application methods, aerosol spray deposition, and thin cladding. The thusly constructed filter capacitor is electrically connected to the terminal pin 116 by a layer of conductive polyimide 119, and the like.

In some applications it may be desirable to increase the capacitor's filtering frequency range, and this is done by increasing the number of segregated active and ground electrode layers supported on the LTCC substrate 102. This means that after a bottom electrode layer is printed and dried, a dielectric layer is printed on top of the as-printed and dried bottom electrode pattern. After the dielectric layer is printed and dried, a top electrode layer is printed on the dried dielectric pattern. A similar print-dry procedure can be followed to print additional function layers until the desired capacitor sets are achieved.

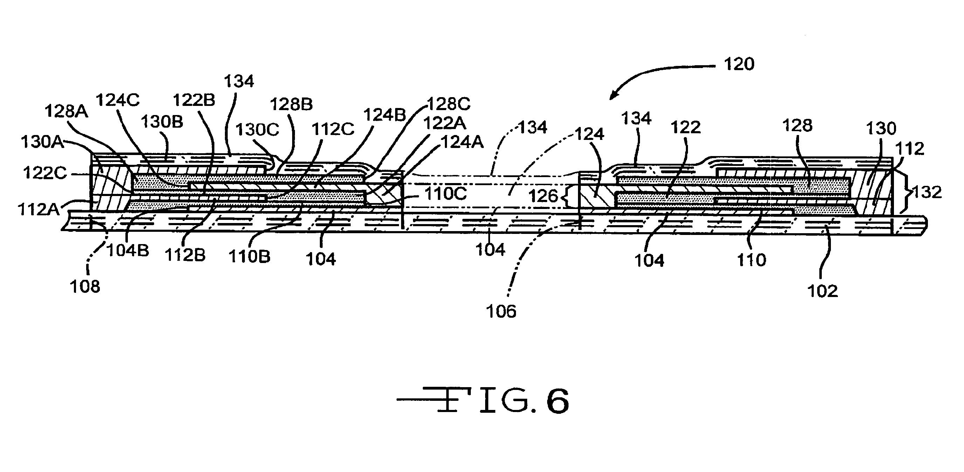

In Fig. 6, a capacitor 120 containing three plate sets is shown. This capacitor 120 is built by screen-printing additional active and ground electrode plates sandwiched around an intermediate dielectric layer on top of the filter capacitor structure 100 illustrated in Figs. 3 to 5, but without the cap LTCC layer 114. In particular, a second dielectric layer 122 is first screen-printed over the bottom ground electrode layer 112. The second dielectric layer 122 has a proximal base portion 122A that begins at the distal edge 110C of the first dielectric layer 110. Dielectric layer 122 is in direct contact with an exposed portion of the distal planar portion 110B of the first dielectric layer and continues to a distal planar portion 122B in direct contact with the upper surface of the distal planar portion 112B of the first ground electrode layer 112. However, the second dielectric layer 122 terminates at an edge 122C spaced from the outer edge 108 of the capacitor 120. This second dielectric layer 122 is completed by drying as previously described.

In an alternate embodiment, the dielectric layer 122 is a laminate of the LTCC tape that is placed on top of the ground electrode layer 112. The dielectric layer 122 is preferably a three layer laminate of LTCC tape.

A second active electrode layer 124 is then screen-printed on top of the second dielectric layer 122. In its finished form, the second active electrode layer 124 has a proximal base portion 124A in direct contact with the first active electrode layer 104 adjacent to the terminal pin bore 106. The direct contact between the proximal base portion 124A of the second active electrode layer 124 forms a common active base 126 having an edge adjacent to the terminal pin bore 106. The proximal base portion 124A leads to a distal planar portion 124B of the second active electrode plate 124 that is in direct contact with the upper surface of the second dielectric layer 122. The distal planar portion 124B of the second active layer 124 extends to an edge 124C that is in vertical alignment with the edge 104B of the first active electrode layer 104. The second active electrode layer 124 is then subjected to a drying process as previously described.

A third dielectric layer 128 is next screen-printed over the second active electrode layer 124. The third dielectric layer 128 has a proximal base portion 128A that begins at the edge 122C of the distal portion 122B of the second dielectric layer 122 and in direct contact therewith, and continues to a distal planar portion 128B in direct contact with the distal planar portion 124B of the second active electrode layer 124. However, the third dielectric layer 128 terminates at an edge 128C spaced from the terminal pin bore 106. The third dielectric layer 128 is completed by drying as previously described. Alternatively, the dielectric layer is a laminate of LTCC tape.

A second ground electrode layer 130 is then screen-printed on top of the third dielectric layer 128. The second ground electrode layer 130 has a proximal base portion 130A in direct contact with the proximal base portion 112A of the first ground electrode layer 112 adjacent to the outer edge 108 of the capacitor 120. The proximal base portion 130A leads to a distal planar portion 130B of the second ground electrode plate 130 that is in direct contact with the upper surface of the third dielectric layer 128. The distal portion 130B extends to an edge 130C that is in vertical alignment with the edge 112C of the first ground electrode layer 112. The direct contact between the proximal base portions 112A, 130A of the respective first and second ground electrodes 112, 130 forms a common ground base 132 having an edge aligned with the capacitor outer edge 108. The second ground electrode layer 130 is then subjected to a drying step as previously described.

This alternating pattern of screen-printing an active layer followed by a dielectric layer followed by a ground electrode layer continues until as many active/dielectric/ground layer sets as are needed to obtain a desired capacitance value and voltage rating. In each set, the proximal ends of the active electrode layers are in direct contact with each other immediately adjacent to the terminal pin bore 106 and the proximal ends of the ground electrode layers are in direct contact with each other immediately adjacent to the outer edge 108 of the capacitor. Every other dielectric layer has its proximal end in direct contact with the distal portion of the dielectric layer immediately below it, alternating first adjacent to the terminal pin bore 106, then adjacent to the outer capacitor edge 108.

The filter capacitor 120 is finished by a cap LTCC layer 134. In its finished form, the cap LTCC layer 134 extends from the outer capacitor edge 108 to the terminal pin bore 106. The cap LTCC layer is of similar materials as the LTCC substrate 102. The capacitor assembly consisting of the LTCC substrate 102/active layers 104, 124/dielectric layers 110, 122, 128/ground electrode layers 112, 130/cap LTCC layer 134 is then subjected to an isostatic pressing at about 3,000 psi for about 10 minutes at from about 65°C to about 85°C. The thusly formed capacitors are individually punched or otherwise cut from the substrate 102 of Fig. 2 and subjected to a final sintering at about 700°C to about 950°C for about 10 to 30 minutes. As is the case with the LTCC substrate 102, the cap LTCC layer 134 preferably consists of three layer of tape, although more or less can be used, if desired.

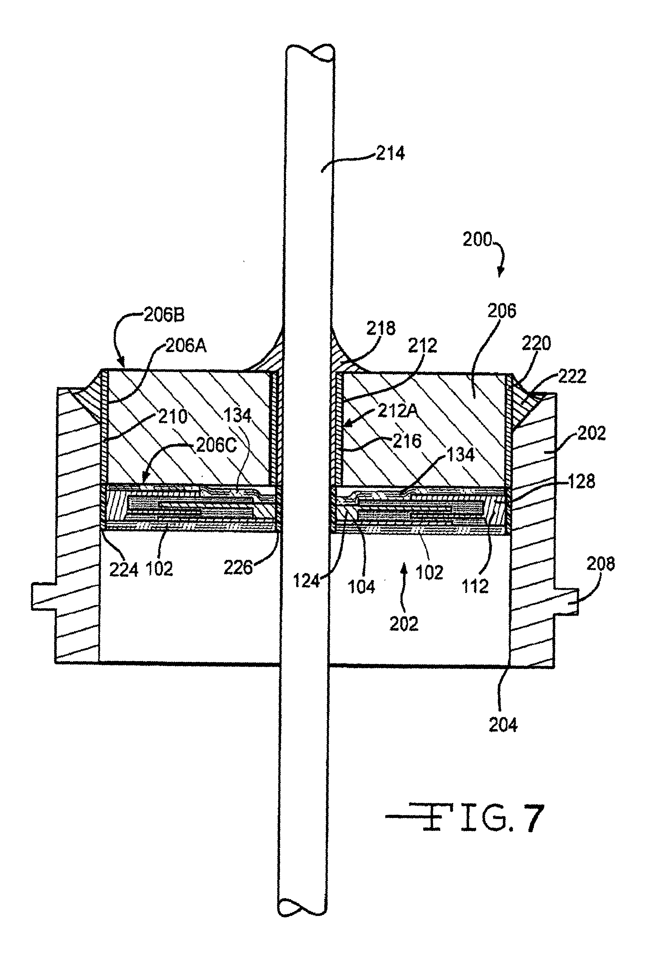

Fig. 7 shows the capacitor 120 of Fig. 6 attached to a feedthrough terminal pin assembly 200. The feedthrough terminal pin assembly 200 comprises a ferrule 202 defining an insulator-receiving bore 204 surrounding an insulator 206. The ferrule includes a surrounding flange 208 to facilitate attachment of the feedthrough capacitor assembly 200 to the casing of, for example, an implantable medical device. The method of attachment may be by laser welding or other suitable methods. The insulator 206 comprises a surrounding sidewall 206A extending to a first upper surface 206B and a second lower surface 206C. A metallization layer 210 is applied to the insulator sidewall 206A to aid in the creation of a brazed hermetic seal. Suitable materials for the ferrule 202, insulator 206 and metallization layer 210 are the same as described for these components with respect to the prior art capacitor 10.

The insulator 206 has a sufficient number of bores 212; in this exemplary feedthrough there is one, to receive the requisite number of terminal pins 214. The inner borc surface 212A is provided with a metallization layer 216 in a similar manner as the previously described insulator sidewall 206A. The terminal pin 214 is hermetically sealed in the bore 212 by a conductive, biostable material 218, such as gold or gold alloy, contacting the metallization layer 216 and the terminal pin 214. Similarly, a metallization layer 220 is provided on the insulator sidewall 206A. A gold braze 222 hermetically seals the insulator metallization 220 to the ferrule 202.

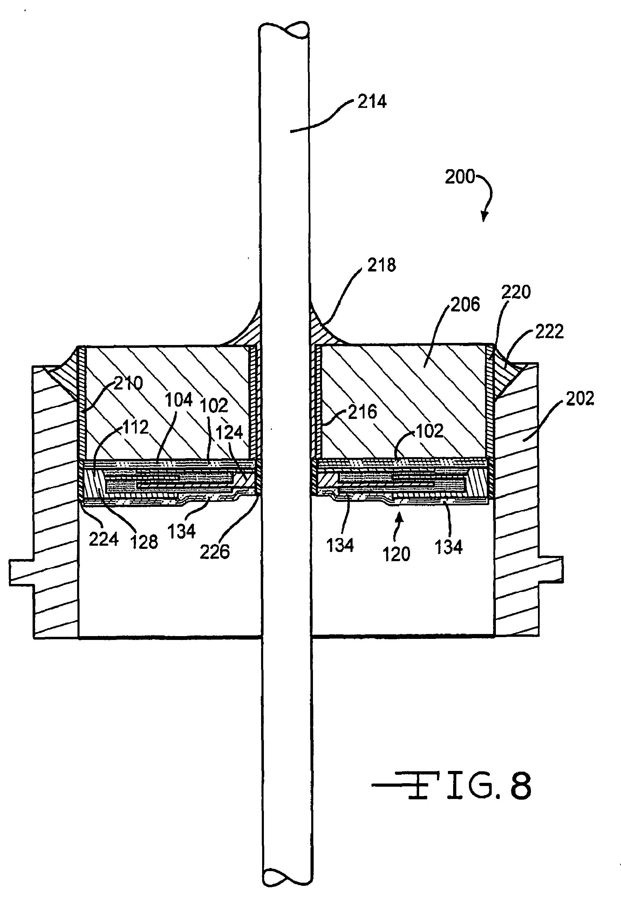

In the exemplary embodiment of Fig. 7, the filter capacitor 120 is attached to the feedthrough terminal pin sub-assembly 202 with the cap LTCC layer 134 seated against the lower insulator surface 206C. A conductive adhesive 224 contacts between the active electrode layers 104, 124 and the terminal pin 214 and a conductive adhesive 226 contacts between the ground electrodes 112, 128 and the ferrule 202. This means that the substrate 102 faces the interior of the medical device housing. In the exemplary embodiment of Fig. 8, the filter capacitor 120 is attached to the feedthrough terminal pin sub-assembly 200 with the LTCC substrate 102 seated against the lower insulator surface 206C and the cap LTCC layer 134 facing the housing interior.

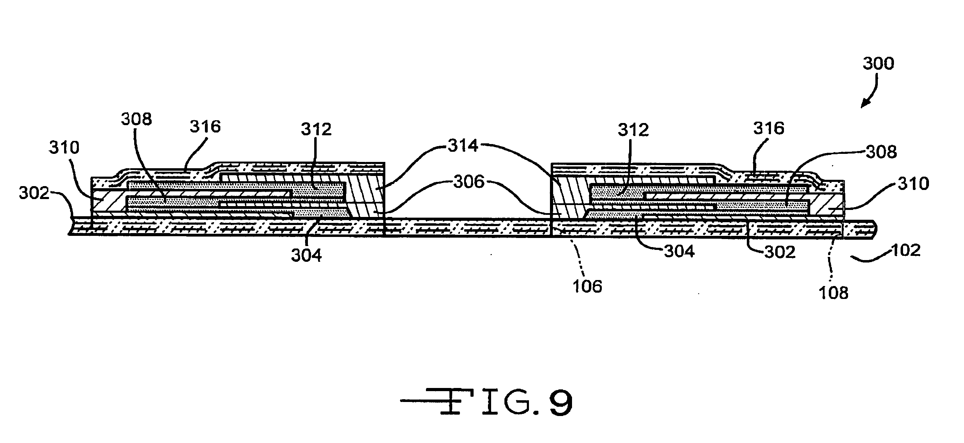

Fig. 9 is a cross-sectional view of another embodiment of a filter capacitor 300 according to the present invention. This filter capacitor 300 is similar in construction to the filter capacitor 120 shown in Fig. 6, except that the first layer screen-printed on top of the LTCC substrate 102 is a first ground electrode layer 302 instead of an active electrode layer. This is followed by a first dielectric layer 304, a first active electrode layer 306, a second dielectric layer 308, a second ground electrode layer 310, a third dielectric layer 312, a second active electrode layer 314 and finally a cap LTCC layer 316.

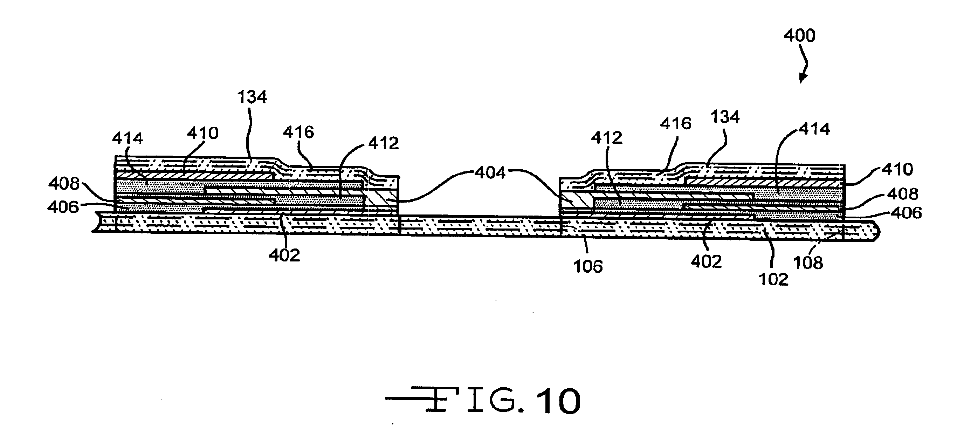

Fig. 10 is a cross-sectional view of another exemplary embodiment of a filter capacitor 400 according to the present invention. Instead of the first and second active electrode layers 402, 404 being in direct physical contact with each other immediately adjacent to the terminal pin bore 106, they are segregated from each other. This is done by extending the first dielectric layer 406 completely to the terminal pin bore 106. Likewise, the first and second ground electrode layers 408, 410 are segregated from each other adjacent to the outer edge 108 of the substrate 102. This is done by extending the second and third dielectric layers 412, 414 to the outer substrate edge 108. A cap LTCC layer 416 is also shown.

Thus, the present invention provides a filter capacitor that is readily attachable to a hermetic feedthrough to provide a filter feedthrough capacitor. The LTCC substrate and cap are of ceramic materials that maintain their shape and structure dimensions even after undergoing sintering. Consequently, the active and ground electrode layers along with the intermediate dielectric layer can be laid down or deposited by a screen-printing technique, which means that they can be made relatively thin. The result is a functional filter capacitor that is as robust as a conventional prior art capacitor made using tape cast technology.

It is appreciated that various modifications to the invention concepts described herein may be apparent to those of ordinary skill in the art without departing from the scope of the present invention as defined by the appended claims.

## Claims

1. A filter feedthrough capacitor, which comprises:
a) a conductive ferrule comprising an annular sidewall having a ferrule opening extending axially along the ferrule sidewall from a first end to a second, opposite end thereof;
b) at least one conductive terminal pin;
c) an insulator having an outer sidewall hermetically sealed to an inner surface of the ferrule sidewall and comprising an insulator opening for passage of the terminal pin there through;
d) a filter capacitor mounted axially at one outer side of the insulator and having a central opening formed therein for pass-through reception of the terminal pin, wherein the capacitor comprises:
i) a planar substrate having an opening for passage of the terminal pin there through and an annular outer edge residing adjacent to the inner surface of the ferrule sidewall;
ii) at least a first electrode layer of an electrically conductive material supported on the substrate having a first edge terminating immediately adjacent to the terminal pin bore;
iii) a first dielectric layer covering at least a portion of the first electrode layer and having opposed ends spaced from the terminal pin bore and the outer edge of the substrate, respectively; and
iv) at least a second, opposite polarity electrode layer of an electrically conductive material supported on the substrate and having a second edge terminating immediately adjacent to the outer edge of the substrate; and
e) a conductive material providing electrical conductivity from one of the first and second electrode layers to the terminal pin and from the other of the first and second electrode layers to the ferrule.

2. The filter feedthrough capacitor of claim 1 wherein the substrate is of at least one first layer of a low temperature co-fired ceramic (LTCC) tape.

3. The filter feedthrough capacitor of claim 2 wherein the LTCC tape includes a ceramic material selected from the group consisting of alumina, zirconia, aluminum nitride, boron nitride, silicon carbide, and mixtures thereof.

4. The filter feedthrough capacitor of claim 2 or claim 3 wherein the LTCC tape includes borosilicate.

5. The filter feedthrough capacitor of any of claims 2 to 4 wherein the LTCC tape includes a dielectric material selected from barium strontium titanate and sodium bismuth titanate.

6. The filter feedthrough capacitor of any of claims 1 to 5 wherein the first and second electrode layers are of Ag-Pt.

7. The filter feedthrough capacitor of any of claims 1 to 6 wherein the dielectric layer is of a dielectric material having a dielectric constant of at least about 10,000 k.

8. The filter feedthrough capacitor of any of claim 1 to 7 wherein at least one layer of a second LTCC tape is supported on the first electrode layer, the dielectric layer and the second electrode layer.

9. The filter feedthrough capacitor of any of claims 1 to 8 further comprising two first electrode layers, the second one having a base portion leading to a distal electrode portion extending towards a substrate edge.

10. The filter feedthrough capacitor of claim 9 wherein the first electrodes are active electrodes and the base portion of the second active electrode layer contacts the first active electrode layer immediately adjacent to the terminal pin bore.

11. The filter feedthrough capacitor of any of claims 1 to 10 wherein the first dielectric layer has a first base portion contacting the substrate and extending towards the terminal pin bore and further comprising a second dielectric layer having a second base portion contacting the first distal portion of the first dielectric layer and leading to a second distal dielectric portion extending towards the outer substrate edge.

12. The filter feedthrough capacitor any of claims 1 to 11 further comprising two second electrode layers, the second one having a base portion leading to a distal electrode portion extending towards an opposite substrate edge.

13. The filter feedthrough capacitor of claim 12 wherein the second electrodes are ground electrodes and the base portion of the second ground electrode layer contacts the first ground electrode layer immediately adjacent to the outer edge of the substrate.

14. The filter feedthrough capacitor of any of claims 1 to 13 wherein the insulator is provided with a metallization layer on the insulator opening and on the outer sidewall and these metallization layers are hermetically sealed to the terminal pin and to the inner surface of the ferrule sidewall, respectively.

15. The filter feedthrough capacitor of any of claims 1 to 14 having a dielectric constant up to about 13,000 k.

16. A filter feedthrough capacitor, which comprises:
a) a conductive ferrule comprising an annular sidewall having a ferrule opening extending axially along the ferrule sidewall from a first end to a second, opposite end thereof;
b) at least one conductive terminal pin;
c) an insulator having an outer sidewall hermetically sealed to an inner surface of the ferrule sidewall and comprising an insulator opening for passage of the terminal pin there through;
d) a filter capacitor mounted axially at one outer side of the insulator and having a central opening formed therein for pass-through reception of the terminal pin, wherein the capacitor comprises:
i) a planar substrate of at least one first layer of a low temperature co-fired ceramic (LTCC) tape having an opening for passage of the terminal pin there through and an annular outer edge residing adjacent to the inner surface of the ferrule sidewall;
ii) at least two first active electrode layers of an electrically conductive material contacting each other adjacent to the terminal pin bore and supported there by the substrate;
iii) a dielectric layer covering at least a portion of each of the first active electrode layers and having opposed ends extending to the terminal pin bore and the outer edge of the substrate; and
iv) at least two second ground electrode layers of an electrically conductive material segregated from the active electrode layers by the dielectric layer, the ground electrode layers contacting each other adjacent to the outer edge of the substrate and supported there by the substrate; and
e) a conductive material providing electrical conductivity from the active electrode layer to the terminal pin and from the ground electrode layer to the ferrule.

17. A filter capacitor, which comprises:
a) a planar substrate of a non-conductive material having an opening for passage of at least one terminal pin there through and an annular outer edge;
b) at least a first active electrode layer of an electrically conductive material supported on the substrate having a first edge terminating immediately adjacent to the terminal pin bore;
c) a first dielectric layer covering at least a portion of the first active electrode layer and having opposed ends spaced from the terminal pin bore and the outer edge of the non-conductive substrate, respectively; and
d) at least a second ground electrode layer of an electrically conductive material supported on the non-conductive substrate and having a second edge terminating immediately adjacent to the outer edge of the non-conductive substrate, wherein the active electrode layer is electrically connectable to a terminal pin and the ground electrode layer is electrically connectable to a conductive substrate.

18. The filter capacitor of claim 17 wherein the non-conductive substrate is of at least one first layer of a low temperature co-fired ceramic (LTCC) tape.

19. The filter capacitor of claim 18 wherein the LTCC tape includes a ceramic material selected from the group consisting of alumina, zirconia, aluminum nitride, boron nitride, silicon carbide, and mixtures thereof.

20. The filter capacitor of claim 18 or claim 19 wherein the LTCC tape includes borosilicate.

21. The filter capacitor of any of claims 18 to 20 wherein the LTCC tape includes a dielectric material selected from barium strontium titanate and sodium bismuth titanate.

22. The filter capacitor of any of claims 17 to 21 wherein the active and ground electrode layers are of Ag-Pt.

23. The filter capacitor of any of claim 17 to 22 wherein the dielectric layer is of a dielectric material having a dielectric constant of at least about 10,000 k.

24. The filter capacitor of any of claims 17 to 23 wherein at least one layer of a second LTCC tape is supported on the first electrode layer, the dielectric layer and the second electrode layer.

25. The filter capacitor of any of claims 17 to 24 further comprising a second active electrode layer having a base portion leading to a distal active electrode portion extending towards the outer edge of the non-conductive substrate, wherein the base portion of the second active electrode layer contacts the first active electrode layer immediately adjacent to the terminal pin bore.

26. The filter capacitor of claim 25 wherein the first dielectric layer has a first base portion contacting the non-conductive substrate and extending towards the terminal pin bore and further comprising a second dielectric layer having a second base portion contacting the first distal portion of the first dielectric layer and leading to a second distal dielectric portion extending towards the outer edge of the non-conductive substrate.

27. The filter capacitor of any of claims 17 to 26 further comprising a second ground electrode layer having a base portion leading to a distal ground electrode portion extending towards the terminal pin bore, wherein the base portion of the second ground electrode layer contacts the first ground electrode layer immediately adjacent to the outer edge of the non-conductive substrate.

28. The filter capacitor of any of claims 17 to 27 adapted for connection to two or more terminal pins.

29. A method for providing filter capacitor assembly, comprising the steps of:
a) providing a planar substrate;
b) screen-printing at least a first electrode layer of an electrically conductive material on the substrate;
c) screen-printing a first dielectric layer covering at least a portion of the first electrode layer and having opposed ends spaced from an intended location of a terminal pin bore and an intended location of an outer edge of the substrate, respectively;
d) screen-printing at least a second, opposite polarity electrode layer of an electrically conductive material on the substrate, the second electrode layer having a second edge terminating immediately adjacent to the outer edge of the substrate; and
e) cutting the substrate to provide an opening for passage of a terminal pin there through and an annular outer edge.

30. The method of claim 29 including providing the substrate being of at least one first layer of a low temperature co-fired ceramic (LTCC) tape.

31. The method of claim 30 including providing the LTCC tape of a ceramic material selected from the group consisting of alumina, zirconia, aluminum nitride, boron nitride, silicon carbide, and mixtures thereof.

32. The method of claim 30 or claim 31 including providing borosilicate in the LTCC tape.

33. The method of any of claims 30 to 32 including providing the LTCC tape of a dielectric material selected from barium strontium titanate and sodium bismuth titanate.

34. The method of any of claims 29 to 33 including providing the active and ground electrode layers being of Ag-Pt.

35. The method of any of claims 29 to 34 including providing the dielectric layer being of a dielectric material having a dielectric constant of at least about 10,000 k.

36. The method of any of claims including supporting at least one layer of a second LTCC tape on the first electrode layer, the dielectric layer and the second electrode layer.

37. The method of any of claims 29 to 36 including drying the first electrode layer screen-printed on the substrate prior to depositing the dielectric layer.

38. The method of claim 37 including drying the first dielectric layer screen-printed on the first electrode layer and the substrate prior to depositing the second electrode layer.

39. The method of claim 38 including drying the second electrode layer screen-printed on the dielectric layer and the substrate.

40. The method of claim 39 including supporting at least one layer of a second LTCC tape on the first electrode layer, the dielectric layer and the second electrode layer.

41. The method of claim 40 including isostatically pressing and sintering the assembly of the second LTCC tape supported on the first and second electrode layers and the intermediate dielectric layer deposited on the substrate.

42. The method of any of claims 29 to 41 including providing a feedthrough terminal pin assembly comprising the further steps of:
a) providing a conductive ferrule comprising an annular sidewall having a ferrule opening extending axially along the ferrule sidewall from a first end to a second, opposite end thereof;
b) providing at least one conductive terminal pin;
c) hermetically sealing an outer sidewall of an insulator to an inner surface of the ferrule sidewall and further providing the insulator comprising an insulator opening for passage of the terminal pin there through;
d) hermetically sealing the terminal pin in the insulator opening;
e) axially mounting the filter capacitor at one outer side of the insulator and further providing the capacitor having a central opening formed therein for pass-through reception of the terminal pin; and
f) providing a first conductive material contacting between the first electrode layer and one of the terminal pin and the ferrule and a second conductive material contacting between the second electrode layer and the other of the terminal pin and the ferrule.

43. The method of any of claims 29 to 42 including providing the first electrode being an active electrode and including further screen-printing a second active electrode layer having a base portion leading to a distal active electrode portion extending towards the outer substrate edge, wherein the base portion of the second active electrode layer contacts the first active electrode layer immediately adjacent to the terminal pin bore.

44. The method of any claims 29 to 43 including providing the first dielectric layer having a first base portion contacting the substrate and a first distal portion extending towards the terminal pin bore and including further screen-printing a second dielectric layer having a second base portion contacting the first distal portion of the first dielectric layer and leading to a second distal dielectric portion extending towards the outer substrate edge.

45. The method of any of claims 29 to 44 wherein the second electrode layer is a ground electrode and including further screen-printing a second ground electrode layer having a base portion leading to a distal ground electrode portion extending towards the terminal pin bore, wherein the base portion of the second ground electrode layer contacts the first ground electrode layer immediately adjacent to the outer edge of the substrate.

46. The method of any of claims 29 to 45 including providing the insulator with a metallization layer on the insulator opening and on the outer sidewall and hermetically sealing these metallization layers to the terminal pin and to the inner surface of the ferrule sidewall, respectively.

47. The method of any of claims 29 to 46 including providing the dielectric layer having a dielectric constant up to about 13,000 k.
